# EUROPEAN PATENT APPLICATION

(11) **EP 4 450 485 A1**
(43) Date of publication of application: **23.10.2024**
(21) Application number: 22907463.8
(22) Date of filing: 14.12.2022
(51) Int. Cl.: C07C 213/10, A61K 9/16, A61K 31/713, A61K 47/18, A61K 47/24, A61K 47/28, A61K 47/34, A61P 35/00, C07C 219/06, C07C 219/08, C07C 227/40, C07C 229/12, C12N 15/113

(54) **METHOD FOR PURIFYING COMPOSITION**

(30) Priority: 16.12.2021 JP 2021204594
(71) Applicant: JSR Corporation, Tokyo 105-8640 (JP); National University Corporation Hokkaido University, Hokkaido 060-0808 (JP)
(72) Inventor: MIYAZAKI Yuta, Tokyo 105-8640 (JP); ARAI Takayuki, Tokyo 105-8640 (JP); ITO Masayoshi, Tokyo 105-8640 (JP); NEGISHI Hashiru, Tokyo 105-8640 (JP); HARASHIMA Hideyoshi, Sapporo-shi, Hokkaido 060-0808 (JP); SATO Yusuke, Sapporo-shi, Hokkaido 060-0808 (JP)
(74) Representative: TBK
(86) International application number: PCT/JP2022/045954
(87) International publication number: WO 2023/112939

(57) **Abstract**

A method for purifying a composition, the method including a step of dissolving a composition including a compound represented by Formula (1) [in Formula (1), R¹ represents -N(R²)-R² (R² represents a C1 to C4 alkyl group), each R³ and R⁴ represents a C3 to C8 alkanediyl group, R⁵ represents a hydroxyl group, R⁶ represents -R⁷-OH (R⁷ represents a C4 to C12 alkanediyl group) or a hydrogen atom, and n represents an integer of 0 or 1] in an aqueous layer to perform liquid-liquid extraction, in which an oil layer used for the liquid-liquid extraction includes one or more kinds of liquids selected from the group consisting of a ketone-based liquid, an ester-based liquid, and an ether-based liquid, all of which have a solubility parameter (SP value) of 14.8 to 20.5 (MPa^{1/2}).

## Description

### [Technical Field]

The present invention relates to a method for purifying a composition. More particularly, the present invention relates to a method for purifying a composition, a composition, a compound, and a lipid nanoparticle. Priority is claimed on Japanese Patent Application No. 2021-204594, filed December 16, 2021, the content of which is incorporated herein by reference.

### [Background Art]

Most of a drug administered to a living body is metabolized in the liver and excreted through the kidney before reaching a target site (on-target) such as a receptor or a gene. In addition, the drug may act on a non-target site (off-target) and cause side effects.

A drug delivery system (DDS) is a drug development technology for controlling in vivo kinetics of a drug in order to maximize the effect of the drug. A drug can be delivered to a target site (on-target) at an appropriate concentration and for an appropriate time period by the DDS.

As the DDS, lipid nanoparticles encapsulating a drug, and the like are known. For example, Patent Document 1 and Non-Patent Document 1 describe lipid nanoparticles encapsulating siRNAs. In addition, it is described in Patent Document 1 and Non-Patent Document 1 that the lipid nanoparticles encapsulating siRNAs are useful for cancer immunotherapy based on knockdown using an siRNA targeting an immunosuppressive factor of a dendritic cell as a target site.

### [Citation List]

### [Patent Document]

### [Patent Document 1]

United States Patent Application, Publication No. 2017/00273905

### [Non-Patent Document]

### [Non-Patent Document 1]

Warashina S., et al., A lipid nanoparticle for the efficient delivery of siRNA to dendritic cells, Journal of Controlled Release, 225, 183-191, 2016.

### [Summary of Invention]

### [Technical Problem]

However, the inventors of the present invention have found that the lipids constituting the lipid nanoparticles described in Patent Document 1 and Non-Patent Document 1 include impurities, and these impurities have adverse effects on the in vivo kinetics of the lipid nanoparticles encapsulating an siRNA to a target site and reduce the knockdown efficiency of a target gene. Thus, an object of the present invention is to provide a technology for removing impurities included in a composition including a lipid.

### [Solution to Problem]

The present invention includes the following embodiments.
[1] A method for purifying a composition, the method including:
   a step of dissolving a composition including a compound represented by the following Formula (1) in an aqueous layer to perform liquid-liquid extraction and refining the compound represented by the following Formula (1),
   in which an oil layer used for the liquid-liquid extraction includes one or more kinds of liquids selected from the group consisting of a ketone-based liquid, an ester-based liquid, and an ether-based liquid, all of which have a solubility parameter (SP value) of 14.8 to 20.5 (MPa^{1/2}), 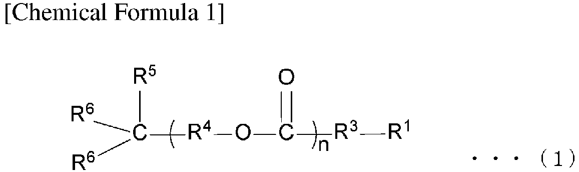 in the Formula (1), R¹ represents -N(R²)-R² (where each R² independently represents a C1 to C4 alkyl group), R³ represents a C3 to C8 alkanediyl group, R⁴ represents a C3 to C8 alkanediyl group, R⁵ represents a hydroxyl group, each R⁶ independently represents -R⁷-OH (where R⁷ represents a C4 to C12 alkanediyl group) or a hydrogen atom, and n represents an integer of 0 or 1.
[2] The method for purifying according to [1], in which the ketone-based liquid is cyclohexanone, methyl isobutyl ketone, or diisopropyl ketone, the ester-based liquid is ethyl acetate or butyl acetate, and the ether-based liquid is diethyl ether, dipropyl ether, cyclopentyl methyl ether, or propylene glycol monomethyl ether acetate.
[3] A composition including:
   one or more kinds of compounds selected from the group consisting of a compound (2-1) in which, in a compound represented by the following Formula (2), n is 0, both R⁹'s are -R⁷-O-C(O)-R¹⁰, and R⁵ is a hydroxyl group,
   a compound (2-2) in which, in a compound represented by the following Formula (2), n is 0, one R⁹ is -R⁷-O-C(O)-R¹⁰, the other R⁹ is -O-C(O)-R¹⁰, and R⁵ is a hydrogen atom, and
   a compound (2-3) in which, in a compound represented by the following Formula (2), n is 1, both R⁹'s are -R⁷-O-C(O)-R¹⁰, and R⁵ is a hydroxyl group,
   in which a total content proportion of the compound (2-1), the compound (2-2), and the compound (2-3) included in the composition is 90% by mass or more, 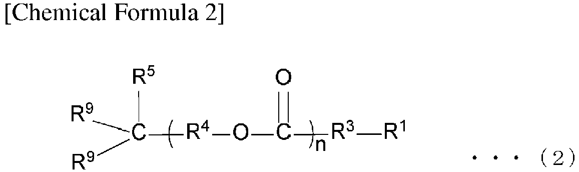 in the Formula (2), R¹ represents -N(R²)-R² (where each R² independently represents a C1 to C4 alkyl group), R³ represents a C3 to C8 alkanediyl group, R⁴ represents a C3 to C8 alkanediyl group, R⁵ represents a hydroxyl group or a hydrogen atom, each R⁹ independently represents -R⁷-O-C(O)-R¹⁰ or -O-C(O)-R¹⁰ (where R⁷ represents a C4 to C12 alkanediyl group, and R¹⁰ represents a C4 to C25 alkenyl group), where at least one R⁹ is -R⁷-O-C(O)-R¹⁰, and n represents an integer of 0 or 1.
[4] The composition according to [3], in which a content proportion of the compound (2-1) included in the composition is 85% to 99% by mass.
[5] The composition according to [3] or [4], in which a total content proportion of the compound (2-2) and the compound (2-3) included in the composition is 0.1% to 15% by mass.
[6] A compound in which, in the Formula (2) according to [3], n is 0, one R⁹ is - R⁷-O-C(O)-R¹⁰, the other R⁹ is -O-C(O)-R¹⁰, and R⁵ is a hydrogen atom.
[7] A compound in which, in Formula (2) according to [3], n is 1, both R⁹'s are - R⁷-O-C(O)-R¹⁰, and R⁵ is a hydroxyl group.
[8] A lipid nanoparticle formed from a compound included in the composition according to any one of [3] to [5] and encapsulating a drug.
[9] The lipid nanoparticle according to [8], in which the drug is an siRNA.

### [Advantageous Effects of Invention]

According to the present invention, a technology can be provided for removing impurities included in a composition including a lipid.

### [Brief Description of Drawings]

FIG. 1 is a diagram showing a reaction of Scheme (1).
FIG. 2 is a diagram showing a reaction of Scheme (II).
FIG. 3 is a diagram showing a reaction of Scheme (III).

### [Description of Embodiments]

Hereinafter, the present invention will be described in more detail by way of embodiments; however, the present invention is not intended to be limited to the following embodiments.

### [Method for purifying composition]

According to an embodiment, the present invention provides a method for purifying a composition, the method including a step of dissolving a composition including a compound represented by Formula (1) in an aqueous layer to perform liquid-liquid extraction, and refining the compound represented by the following Formula (1), in which an oil layer used for the liquid-liquid extraction includes one or more kinds of liquids selected from the group consisting of a ketone-based liquid, an ester-based liquid, and an ether-based liquid, all of which have a solubility parameter (SP value) of 14.8 to 20.5 (MPa^{1/2}).

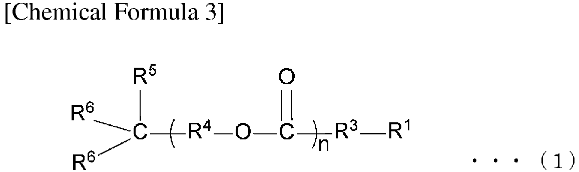

In Formula (1), R¹ represents -N(R²)-R² (here, each R² independently represents a C1 to C4 alkyl group), R³ represents a C3 to C8 alkanediyl group, R⁴ represents a C3 to C8 alkanediyl group, R⁵ represents a hydroxyl group, R⁶ represents -R⁷-OH (here, R⁷ represents a C4 to C12 alkanediyl group) or a hydrogen atom, and n represents an integer of 0 or 1.

As described above, the present inventors have found that impurities are included in the lipid constituting the lipid nanoparticles described in Patent Document 1 and Non-Patent Document 1, and these impurities have adverse effects on the in vivo kinetics of lipid nanoparticles encapsulating an siRNA to a target site and reduce the knockdown efficiency of a target gene. It is difficult to identify the chemical structures of these impurities, and the chemical structures are still unknown.

However, according to the purification method of the present embodiment, the above-described compound represented by Formula (1) can be refined to remove impurities, and a composition (lipid) that will be described later can be produced. In a case where lipid nanoparticles encapsulating an siRNA are produced using this lipid, the in vivo kinetics of the lipid nanoparticles to a target site can be improved, and a decrease in the knockdown efficiency of a target gene can be suppressed, as compared with a case where the purification method of the present embodiment is not performed. In the present specification, improving the in vivo kinetics means that a proportion of the lipid nanoparticles to be delivered to a target site among the administered lipid nanoparticles is increased.

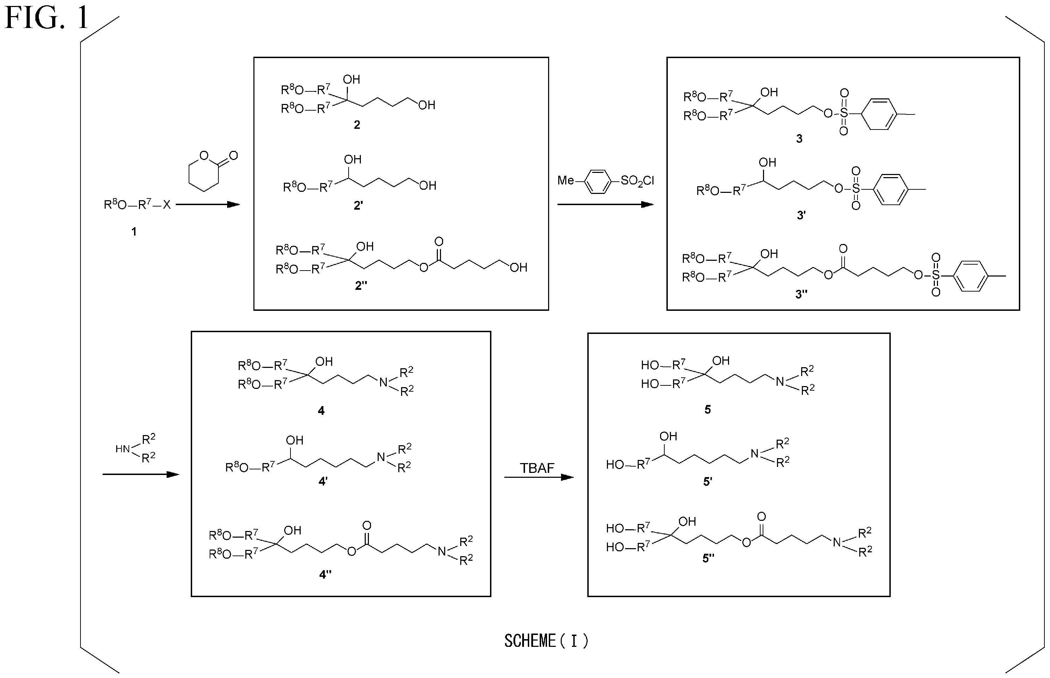

A composition including the above-described compound represented by Formula (1) can be obtained by, for example, a reaction of Scheme (1) shown in FIG. 1. In FIG. 1, R² and R⁷ are the same as those in the above-described Formula (1), each R² independently represents a C1 to C4 alkyl group, each R⁷ independently represents a C4 to C12 alkanediyl group, and R⁸ represents a protective group. In FIG. 1, compounds 2', 2", 3', 3", 4', 4", 5', and 5" are unintended products; however, the present inventors have verified that even though these compounds are present, when the composition (lipid) that will be described below is produced and then lipid nanoparticles encapsulating an siRNA are produced, there are no adverse effects on the in vivo kinetics of the lipid nanoparticles to a target site and on the knockdown efficiency of a target gene.

As the protective group for R⁸, a group which is conventionally used as a protective group for a hydroxyl group can be appropriately used. Specific examples thereof include a tert-butyldimethylsilyl group, a trimethylsilyl group, a triethylsilyl group, a benzyl group, a tert-butyl group, a methoxymethyl group, a 2-tetrahydropyranyl group, an acetyl group, and a benzoyl group.

Liquid-liquid extraction is a separation and thickening method utilizing the partitioning of a solute between two liquids that are immiscible with each other. In the purification method of the present embodiment, a composition including the above-described compound represented by Formula (1) is dissolved in an aqueous layer, and extraction between this aqueous layer and an oil layer that is immiscible with the aqueous layer is performed. As the oil layer to be used for the liquid-liquid extraction, one or more kinds of liquids selected from the group consisting of a ketone-based liquid, an ester-based liquid, and an ether-based liquid, all of which have a solubility parameter (SP value) of 14.8 to 20.5 (MPa^{1/2}), are used. The liquid-liquid extraction can be performed under ordinary conditions. Specifically, for example, the above-described aqueous layer and oil layer are mixed at room temperature, subsequently the mixture is left to stand to separate the aqueous layer and the oil layer, and the aqueous layer is collected. As a result, the above-described compound represented by Formula (1), from which impurities have been removed, can be obtained in the collected aqueous layer.

Here, examples of the ketone-based liquid include cyclohexanone, methyl isobutyl ketone, and diisopropyl ketone. Examples of the ester-based liquid include ethyl acetate and butyl acetate. Examples of the ether-based liquid include diethyl ether, dipropyl ether, cyclopentyl methyl ether, and propylene glycol monomethyl ether acetate. In the present specification, a liquid having both of a -C(O)- group and an ether bond, such as propylene glycol monomethyl ether acetate, is classified into an ether-based liquid, as described above. These liquids may be used singly or may be used as mixtures of two or more kinds thereof.

### [Composition]

According to an embodiment, the present invention provides a composition including: one or more kinds of compounds selected from the group consisting of a compound (2-1) in which, in a compound represented by Formula (2), n is 0, both R⁹'s are -R⁷-O-C(O)-R¹⁰, and R⁵ is a hydroxyl group, a compound (2-2) in which, in a compound represented by Formula (2), n is 0, one R⁹ is -R⁷-O-C(O)-R¹⁰, the other R⁹ is - O-C(O)-R¹⁰, and R⁵ is a hydrogen atom, and a compound (2-3) in which, in a compound represented by Formula (2), n is 1, both R⁹'s are -R⁷-O-C(O)-R¹⁰, and R⁵ is a hydroxyl group, in which a total content proportion of the compound (2-1), the compound (2-2), and the compound (2-3) included in the composition is 90% by mass or more.

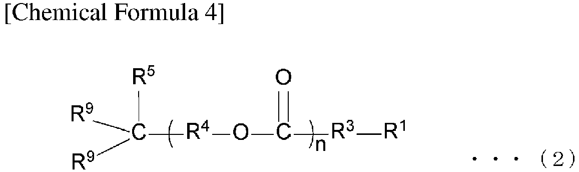

In Formula (2), R¹ represents -N(R²)-R² (here, each R² independently represents a C1 to C4 alkyl group); R³ represents a C3 to C8 alkanediyl group; R⁴ represents a C3 to C8 alkanediyl group; R⁵ represents a hydroxyl group or a hydrogen atom; each R⁹ independently represents -R⁷-O-C(O)-R¹⁰ or -O-C(O)-R¹⁰ (here, R⁷ represents a C4 to C12 alkanediyl group, and R¹⁰ represents a C4 to C25 alkenyl group), where at least one R⁹ is -R⁷-O-C(O)-R¹⁰; and n represents an integer of 0 or 1.

General formulas of the compound (2-1), compound (2-2), and compound (2-3) are shown below. 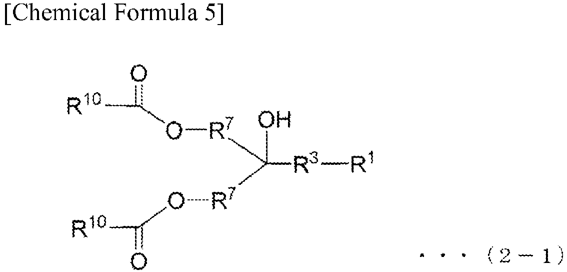 In Formula (2-1), R¹ represents -N(R²)-R² (here, each R² independently represents a Cl to C4 alkyl group); R³ represents a C3 to C8 alkanediyl group; each R⁷ independently represents a C4 to C12 alkanediyl group; and each R¹⁰ independently represents a C4 to C25 alkenyl group. 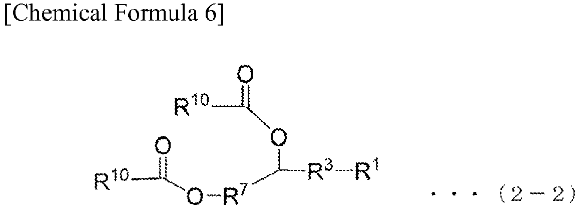 In Formula (2), R¹ represents -N(R²)-R² (here, each R² independently represents a C1 to C4 alkyl group); R³ represents a C3 to C8 alkanediyl group; R⁷ represents a C4 to C12 alkanediyl group; and each R¹⁰ independently represents a C4 to C25 alkenyl group. 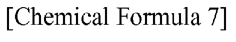 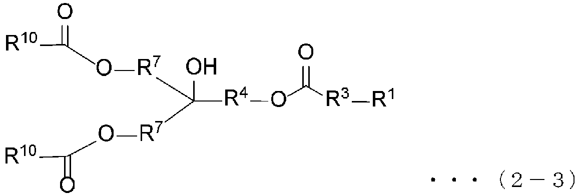 In Formula (2-3), R¹ represents -N(R²)-R² (here, each R² independently represents a C1 to C4 alkyl group); R³ represents a C3 to C8 alkanediyl group; R⁴ represents a C3 to C8 alkanediyl group; each R⁷ independently represents a C4 to C12 alkanediyl group; and each R¹⁰ independently represents a C4 to C25 alkenyl group.

The composition of the present embodiment is a lipid, and lipid nanoparticles encapsulating a drug can be produced therefrom. The composition of the present embodiment can be obtained by converting R⁶ in the composition including the above-described compound represented by Formula (1), which has been purified by the above-mentioned purification method, into R⁹ (here, R⁹ represents -R⁷-O-C(O)-R¹⁰ or -O-C(O)-R¹⁰). Here, R⁹ represents -R⁷-O-C(O)-R¹⁰ or -O-C(O)-R¹⁰.

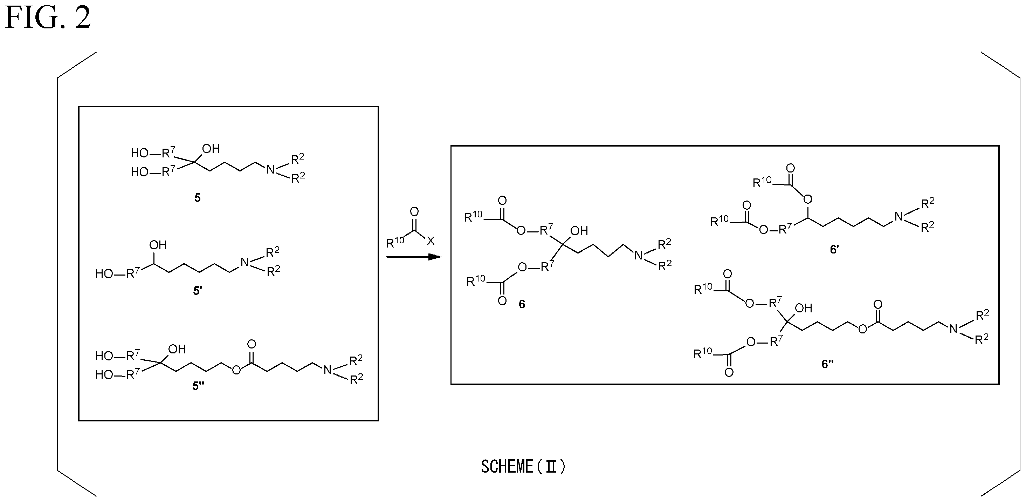

The composition of the present embodiment can be obtained by, for example, a reaction of Scheme (II) shown in FIG. 2. The Scheme (II) is a reaction subsequent to the Scheme (1) shown in FIG. 1. In FIG. 2, R² and R⁷ are the same as those in the above-described Formula (1), each R² independently represents a C1 to C4 alkyl group, and each R⁷ independently represents a C4 to C12 alkanediyl group. Each R¹⁰ independently represents a C4 to C25 alkenyl group.

In FIG. 2, compound 6 is an example of the above-described compound (2-1), compound 6' is an example of the above-described compound (2-2), and compound 6" is an example of the above-described compound (2-3). Here, the compounds 6' and 6" are unintended products; however, the present inventors have verified that even though these compounds are present, when lipid nanoparticles encapsulating an siRNA are produced, there are no adverse effects on the in vivo kinetics of the lipid nanoparticles to a target site and on the knockdown efficiency of a target gene.

In FIG. 2, R⁶ is converted to R⁹ (here, R⁹ represents -R⁷-O-C(O)-R¹⁰ or -O-C(O)-R¹⁰) by reacting the composition including the compounds 5, 5', and 5" with X-C(O)-R¹⁰ (here, X is a halogen atom).

The composition of the present embodiment includes one or more kinds of compounds selected from the group consisting of the above-described compounds (2-1), (2-2), and (2-3), and the total content proportion of the compound (2-1), the compound (2-2), and the compound (2-3) included in the composition is 90% by mass or more, preferably 95% by mass or more, more preferably 98% by mass or more, and even more preferably 99% by mass or more, while the balance includes impurities. The composition of the present embodiment can be obtained by carrying out the above-described method for purifying a composition and removing impurities.

In the composition of the present embodiment, the content proportion of the above-described compound (2-1) included in the composition is preferably 85% to 99% by mass. In addition, the total content proportion of the above-described compound (2-2) and the above-described compound (2-3) included in the composition is preferably 0.1% to 15% by mass.

### [Compound]

According to embodiment, the present invention provides a compound (2-2) in which, in the above-described Formula (2), n is 0, one R⁹ is -R⁷-O-C(O)-R¹⁰, the other R⁹ is -O-C(O)-R¹⁰, and R⁵ is a hydrogen atom. The general formula of the compound (2-2) is as described above.

According to another embodiment, the present invention provides a compound (2-3) in which, in the above-described Formula (2), n is 1, both R⁹'s are -R⁷-O-C(O)-R¹⁰, and R⁵ is a hydroxyl group. The general formula of the compound (2-3) is as described above.

### [Lipid nanoparticle]

According to an embodiment, the present invention provides a lipid nanoparticle that is formed from a composition including one or more kinds of compounds selected from the group consisting of the above-described compounds (2-1), (2-2), and (2-3), in which the total content proportion of the compound (2-1), the compound (2-2), and the compound (2-3) included in the composition is 90% by mass or more, preferably 95% by mass or more, more preferably 98% by mass or more, and even more preferably 99% by mass or more, and encapsulates a drug.

The lipid nanoparticle according to the present specification refers to a particle containing a lipid as a main component and having a particle diameter of 10 nm to 1,000 nm. The lipid nanoparticle is also referred to as "Lipid Nanoparticle" (LNP).

In the lipid nanoparticle of the present embodiment, since the incorporation of impurities with adverse effects is reduced, the in vivo kinetics to a target site is favorable. In addition, in a case where the drug is an siRNA, the knockdown efficiency of a target gene is high. The lipid nanoparticle of the present embodiment can be formed from a composition from which impurities have been removed by carrying out the above-described method for purifying a composition.

As the lipid component of the lipid nanoparticle, only one or more kinds of compounds selected from the group consisting of the above-described compounds (2-1), (2-2), and (2-3) may be used; however, generally, for example, one or more kinds of lipids selected from the group consisting of a phospholipid, a glycolipid, a sterol, a saturated or unsaturated fatty acid, and a saturated or unsaturated fatty acid ester are combined to form the lipid nanoparticle. A combination of a plurality of lipids and blending proportions thereof can be appropriately adjusted according to the purpose.

Examples of the phospholipid and phospholipid derivatives include phosphatidylethanolamine, phosphatidylcholine, phosphatidylserine, phosphatidylinositol, phosphatidylglycerol, cardiolipin, sphingomyelin, ceramide phosphorylethanolamine, ceramide phosphorylglycerol, ceramide phosphorylglycerol phosphate, 1,2-dimyristoyl-1,2-deoxyphosphatidylcholine, distearoylphosphatidylcholine, plasmalogen, and phosphatidic acid, and these can be used singly or in combination of two or more kinds thereof. The fatty acid residue in these phospholipids is not particularly limited; however, examples thereof include a saturated or unsaturated fatty acid residue having 12 to 20 carbon atoms, and specific examples include acyl groups derived from fatty acids such as lauric acid, myristic acid, palmitic acid, stearic acid, oleic acid, and linoleic acid. In addition, phospholipids derived from natural substances, such as egg yolk lecithin and soy lecithin, can also be used.

Examples of the glycolipid include glyceroglycolipids (for example, sulfoxyribosyl glyceride, diglycosyl diglyceride, digalactosyl diglyceride, galactosyl diglyceride, and glycosyl diglyceride) and sphingoglycolipids (for example, galactosyl cerebroside, lactosyl cerebroside, and ganglioside).

Examples of the sterol include animal-derived sterols (for example, cholesterol, cholesterol succinic acid, lanosterol, dihydrolanosterol, desmosterol, and dihydrocholesterol), plant-derived sterols (phytosterols) (for example, stigmasterol, sitosterol, campesterol, and brassicasterol), and microbial-derived sterols (for example, zymosterol and ergosterol).

Examples of the saturated or unsaturated fatty acid include saturated or unsaturated fatty acids having 12 to 20 carbon atoms, such as palmitic acid, oleic acid, stearic acid, arachidonic acid, and myristic acid.

Examples of the saturated or unsaturated fatty acid ester include glycerin fatty acid esters in which one or two hydroxyl groups of a glycerol are ester-bonded to a fatty acid. Examples of the fatty acid residue in the glycerin fatty acid ester include acyl groups derived from saturated or unsaturated fatty acids having 12 to 20 carbon atoms, such as palmitic acid, oleic acid, stearic acid, arachidonic acid, and myristic acid. Specific examples thereof include dimyristoyl glycerol (DMG) and distearoyl glycerol (DSG).

A method for producing lipid nanoparticles is not particularly limited. For example, lipid nanoparticles can be produced by dissolving all lipid components in an organic solvent such as chloroform, subsequently performing vacuum drying with an evaporator or spray drying with a spray dryer to form a lipid membrane, subsequently adding a water-based solvent to the above-described dried mixture, and further performing emulsification using an emulsifier such as a homogenizer, an ultrasonic emulsifier, a high-pressure jet emulsifier, or the like. In addition, the lipid nanoparticles can also be produced by, for example, a reverse-phase evaporation method. In a case where it is desirable to control the size of the lipid nanoparticles, extrusion (extrusion filtration) may be performed under high pressure using a membrane filter having a uniform pore diameter, or the like.

The size of the lipid nanoparticles in a dispersed state can be appropriately selected according to the purpose, and for example, the size can be an average particle size of about 60 to 140 nm, for example, an average particle size of about 80 to 120 nm, or for example, an average particle size of about 20 to 50 nm. The particle diameter can be measured by, for example, a dynamic light scattering (DLS) method. In the present specification, the average particle size of the lipid nanoparticles means a number-average particle size measured by DLS. The measurement by DLS can be performed by a routine method using a commercially available DLS apparatus or the like. The polydispersity index (PDI) is about 0.05 to 0.1, preferably about 0.06 to 0.08, and more preferably about 0.07.

The composition of the water-based solvent (dispersion medium) is not particularly limited, and examples thereof include buffer solutions such as a phosphate buffer solution, a citrate buffer solution, and a phosphate-buffered saline; physiological saline; and media for cell culture. These water-based solvents (dispersion media) can stably disperse the lipid nanoparticles; however, sugars (aqueous solutions), including monosaccharides such as glucose, galactose, mannose, fructose, inositol, ribose, and xylose; disaccharides such as lactose, sucrose, cellobiose, trehalose, and maltose; trisaccharides such as raffinose and melezitose; polysaccharides such as cyclodextrin; and sugar alcohols such as erythritol, xylitol, sorbitol, mannitol, and maltitol, and polyhydric alcohols (aqueous solutions) such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether, and 1 ,3-butylene glycol, may be further added. In order to stably store the lipid nanoparticles dispersed in a water-based solvent for a long period of time, from the viewpoint of physical stability such as suppression of aggregation, it is preferable to exclude an electrolyte in the water-based solvent as much as possible. In addition, from the viewpoint of chemical stability of the lipid, it is preferable to set the pH of the water-based solvent to weak acidity to near neutrality (approximately pH 3.0 to 8.0), and it is preferable to remove dissolved oxygen by nitrogen bubbling or the like.

In a case where the obtained aqueous dispersion of lipid nanoparticles is freeze-dried or spray-dried, it may be possible to improve stability by using sugars (aqueous solutions), including monosaccharides such as glucose, galactose, mannose, fructose, inositol, ribose, and xylose; disaccharides such as lactose, sucrose, cellobiose, trehalose, and maltose; trisaccharides such as raffinose and melezitose, polysaccharides such as cyclodextrin; and sugar alcohols such as erythritol, xylitol, sorbitol, mannitol, and maltitol. In addition, in a case where the above-described aqueous dispersion is frozen, it may be possible to improve stability, for example, when the sugars or polyhydric alcohols (aqueous solutions) such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether, and 1,3-butylene glycol are used.

With regard to the lipid nanoparticles of the present embodiment, the drug encapsulated therein is not particularly limited. For example, nucleic acids such as siRNA, microRNA, mRNA, and a plasmid, as well as optional pharmaceutically active ingredients such as an antitumor agent, an anti-inflammatory agent, an antibacterial agent, and an antiviral agent, and optional substances such as a sugar, a peptide, a low-molecular-weight compound, and a metal compound, can be encapsulated in the lipid nanoparticles. These may be encapsulated singly, or a mixture of two or more kinds thereof may be encapsulated.

A small interfering RNA (siRNA) is a low-molecular-weight double-stranded RNA having 21 to 23 base pairs, and the siRNA is involved in RNA interference (RNAi) and suppresses the expression of a gene in a sequence-specific manner by destroying mRNA. Since a gene can be knocked down by RNA interference using siRNA, the siRNA is expected to be applied to the utilization as a medicine and to the field of treatment of cancer and the like. The type of siRNA that can be used is not particularly limited, and any siRNA capable of causing RNA interference may be used; however, generally, a double-stranded RNA having 21 to 23 base pairs and having a structure in which the 3' portion of the RNA chain adopts a structure of protruding by 2 bases, and each chain has a phosphate group at the 5'-end and a hydroxyl group at the 3'-end, can be used as the siRNA. In addition, an siRNA in which the hydroxyl group at the 2'-position of the ribose skeleton has been substituted with a methoxy group, a fluoro group, or a methoxyethyl group, and some phosphodiester bonds have been substituted with phosphorothioate bonds, are also included.

The form of the lipid nanoparticles of the present embodiment is not particularly limited, and examples thereof include a form of being dispersed in a water-based solvent (for example, water, physiological saline, or phosphate-buffered saline), and a form of being freeze-dried from an aqueous dispersion.

### [Examples]

Next, the present invention will be described in more detail by way of Examples; however, the present invention is not intended to be limited to the following Examples. All animal experiments were performed in accordance with a protocol reviewed by the Institutional Animal Care and Use Committee of Hokkaido University and approved by the President of Hokkaido University. In addition, the structures of the compounds included in the composition were identified by ¹H NMR (manufactured by Bruker Corporation, AVANCE III 600).

### [Experimental Example 1]

### (Preparation of composition before purification)

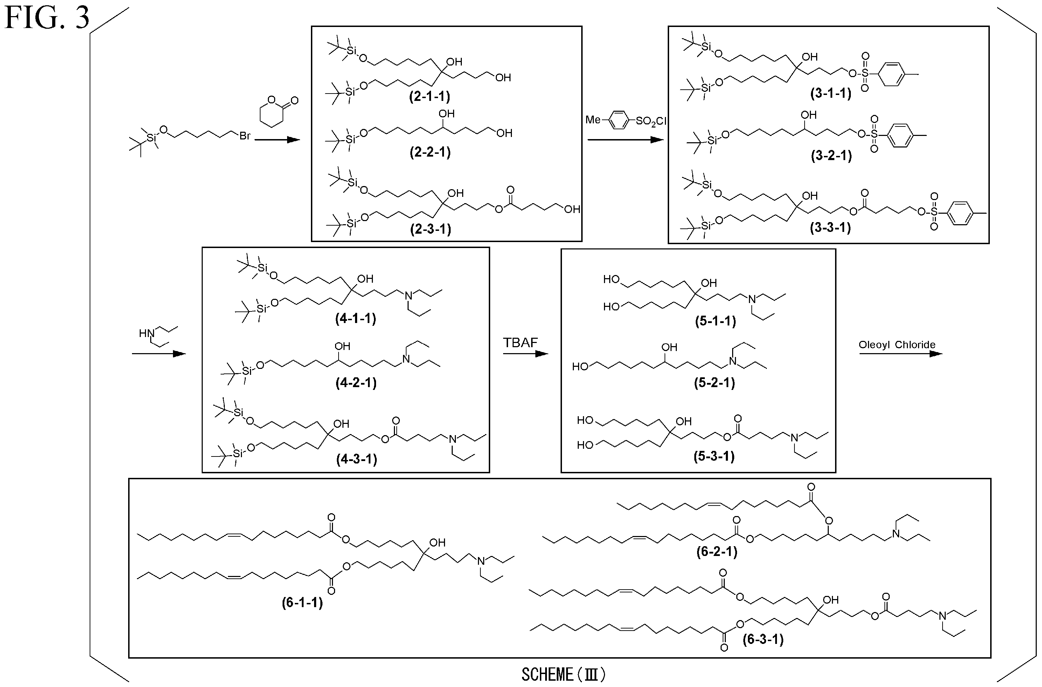

A composition before purification (composition (1-1)) including compound (4-1-1), compound (4-2-1), and compound (4-3-1) was prepared according to the Scheme (III) shown in FIG. 3.

### [Experimental Example 1-1]

### (Preparation of composition including compound (2-1-1), compound (2-2-1), and compound (2-3-1))

26.0 g (88.0 mmol) of (6-bromohexyloxy)-tert-butyldimethylsilane was dissolved in 26 mL of dry diethyl ether to prepare a solution. 1 mL of the above-described solution, 4 mL of dry diethyl ether, and 2.35 g (96.8 mmol) of magnesium shavings were added to a flask in an argon atmosphere, and subsequently a piece of iodine was added thereto. The solution was left to stand at room temperature until the entire solution turned brown, subsequently the solution was heated to 40°C using an oil bath while being stirred, decolorization was checked, and then the above-described (6-bromohexyloxy)-tert-butyldimethylsilane solution was added dropwise to the flask. The reaction was carried out at 40°C for 2 hours, and then the reaction solution was cooled in ice. Subsequently, 3.67 mL (39.6 mmol) of δ-valerolactone dissolved in 3.67 mL of dry diethyl ether was added dropwise to the flask, and the mixture was allowed to react overnight at room temperature. The reaction solution was cooled in ice, diethyl ether was added thereto to dilute the reaction solution, and remaining magnesium was dissolved by adding a saturated aqueous solution of citric acid dropwise to the flask. The organic layer was separated and washed using water and saturated brine. Subsequently, the organic layer was dewatered by adding anhydrous sodium sulfate. This organic layer was filtered, and then the solvent was distilled off using a rotary evaporator to obtain a composition (composition of Experimental Example 1-1) including compound (2-1-1), compound (2-2-1), and compound (2-3-1). The content proportions of the compound (2-1-1), the compound (2-2-1), and the compound (2-3-1) included in the composition (1-1) were such that compound (2-1-1):compound (2-2-1):compound (2-3-1) = 99.0:0.2:0.8.

### [Experimental Example 1-2]

### (Preparation of composition (composition (1-2)) including compound (3-1-1), compound (3-2-1), and compound (3-3-1))

14.0 g of the composition of Experimental Example 1-1 was dissolved in 50 mL of dichloromethane in a flask, 321 mg (2.63 mmol) of N,N-dimethyl-4-aminopyridine (DMAP) and 5.50 mL (39.5 mmol) of diisopropylethylamine were added thereto, and the mixture was cooled in ice. 6.02 g (31.6 mmol) of p-toluenesulfonyl chloride was gradually added to the flask, and then the mixture was allowed to react overnight at room temperature. The solvent was distilled off using a rotary evaporator, subsequently the residue was suspended in ethyl acetate, and the suspension was separated and washed using water and saturated brine. Anhydrous sodium sulfate was added to the organic layer to dewater the organic layer, this mixture was filtered, subsequently the solvent was distilled off using a rotary evaporator, and a composition (composition of Experimental Example 1-2) including compound (3-1-1), compound (3-2-1), and compound (3-3-1).

### [Experimental Example 1-3]

### (Preparation of composition (composition (1-3)) including compound (4-1-1), compound (4-2-1), and compound (4-3-1))

30 mL of tetrahydrofuran was added to 12.4 g of the composition of Experimental Example 1-2 in a flask, and the mixture was cooled to 4°C. Subsequently, 7.38 mL (54.0 mmol) of dipropylamine was added to the flask, and then the mixture was allowed to react at room temperature for 11 days. The solvent was distilled off using a rotary evaporator, subsequently the residue was suspended in ethyl acetate, and the suspension was separated and washed using a 0.5 N aqueous solution of sodium hydroxide and saturated brine. Anhydrous sodium sulfate was added to the organic layer to dewater the organic layer. This organic layer was filtered, subsequently the solvent was distilled off using a rotary evaporator, and a composition (composition of Experimental Example 1-3) including compound (4-1-1), compound (4-2-1), and compound (4-3-1) was obtained.

### [Experimental Example 1-4]

### (Preparation of composition including compound (5-1-1), compound (5-2-1), and compound (5-3-1))

2.23 mL (39 mmol) of acetic acid and 26 mL of a 1.0 M tetrahydrofuran solution of tetrabutylammonium fluoride (TBAF) were added to 7.27 g of the composition of Experimental Example 1-3 in a flask, and the mixture was allowed to react overnight at room temperature. The solvent was distilled off using a rotary evaporator, and a composition (composition (1-1)) including compound (5-1-1), compound (5-2-1), and compound (5-3-1) was obtained.

### [Experimental Example 2]

### (Preparation of composition before purification (composition (1-2))

A composition including the compound (2-1-1), the compound (2-2-1), and the compound (2-3-1) at content proportions of compound (2-1-1):compound (2-2-1):compound (2-3-1) = 92.5:7.0:0.5 was obtained by an operation similar to that of Experimental Example 1-1, except that 3.34 mL (36.0 mmol) of δ-valerolactone was used. Subsequently, a composition (1-2) was obtained by operations similar to those of Experimental Example 1-2, Experimental Example 1-3, and Experimental Example 1-4.

### [Experimental Example 3]

### (Preparation of composition before purification (composition (1-3))

A composition including the compound (2-1-1), the compound (2-2-1), and the compound (2-3-1) at content proportions of compound (2-1-1):compound (2-2-1):compound (2-3-1) = 85.0:0.0:15.0 was obtained by an operation similar to that of Experimental Example 1-1, except that 4.04 mL (43.6 mmol) of δ-valerolactone was used. Subsequently, a composition (1-3) was obtained by operations similar to those of Experimental Example 1-2, Experimental Example 1-3, and Experimental Example 1-4.

### [Experimental Example 4]

### (Purification of composition)

Each of the compositions including the compound (5-1-1), the compound (5-2-1), and the compound (5-3-1) (composition (1-1), composition (1-2), and composition (1-3)) (each 9.5 g) was dissolved in a 0.5 N aqueous solution of hydrochloric acid (150 mL), a solvent (100 mL) as described in Table 1 was added to each of the compositions, and the mixture was stirred for 10 minutes. Subsequently, liquid separation was performed using a liquid separatory funnel, and the aqueous layer was collected. In this case, the liquid separation and washing was repeated until the impurities included in the organic layer disappeared or there was no change in a TLC analysis. Thereafter, a 5M aqueous solution of sodium hydroxide (30 mL) was introduced into the collected aqueous layer, and the mixture was stirred for 10 minutes. Ethyl acetate (100 mL) was further added thereinto, and the mixture was stirred for 10 minutes. Thereafter, the solution was separated using a liquid separatory funnel, and the organic layer was collected. The collected organic layer was separated and washed once using saturated brine. Anhydrous sodium sulfate was added to the organic layer to dewater the organic layer. This mixture was filtered, subsequently the solvent was distilled off using a rotary evaporator, and a purified composition was obtained.

**[Table 1]**

| Solvent | Solubility parameter (MPa^{1/2}) | Name of composition before purification | Name of composition after purification |
|---|---|---|---|
| Cyclohexanone | 20.5 | Composition (1-1) | Composition (2-1) |
| Methyl isobutyl ketone | 169 | Composition (1-2) | Composition (2-2) |
| Diisopropyl ketone | 16.6 | Composition (1-3) | Composition (2-3) |
| Ethyl acetate | 17.9 | Composition (1-1) | Composition (2-4) |
| Butyl acetate | 17.8 | Composition (1-3) | Composition (2-5) |
| Diethyl ether | 14.8 | Composition (1-2) | Composition (2-6) |
| Dipropyl ether | 15.5 | Composition (1-2) | Composition (2-7) |
| Cyclopentyl methyl ether | 17.6 | Composition (1-1) | Composition (2-8) |
| Propylene glycol monomethyl ether acetate | 19.5 | Composition (1-3) | Composition (2-9) |
| Hexane | 14.2 | Composition (1-2) | Composition (2-10) |
| 2-Butanol | 22.1 | Composition (1-3) | Composition (2-11) |

### [Experimental Example 5]

### (Production of composition including compound (6-1-1), compound (6-2-1), and compound (6-3-1))

Compositions (composition (3-1) to composition (3-11)) including compound (6-1-1), compound (6-2-1), and compound (6-3-1) were produced according to the Scheme (111) shown in FIG. 3.

### [Experimental Example 5-1]

### (Production of composition (3-1) to composition (3-11))

Each of the compositions (composition (2-1) to composition (2-11)) purified in Experimental Example 4 was dissolved in 5 mL of dichloromethane in a flask. Subsequently, 900 mg (3.0 mmol) of oleyl chloride was added thereto, and the mixture was cooled to 4°C. 697 µL (5.0 mmol) of triethylamine (TEA) was added dropwise to the flask, and the mixture was reacted at room temperature for 3 hours. The solvent was distilled off using a rotary evaporator, subsequently the residue was suspended in ethyl acetate, and insolubles were removed by filtration. The filtrate was separated and washed using a 0.5 N aqueous solution of sodium hydroxide and saturated brine. Anhydrous sodium sulfate was added to the organic layer to dewater the organic layer. This mixture was filtered, the solvent was distilled off using a rotary evaporator, and a crude product was obtained. The crude product was refined by silica gel chromatography (elution solvent was a continuous gradient of dichloroethane and ethanol), and a composition including compound (6-1-1), compound (6-2-1), and compound (6-3-1) (each of composition (3-1) to composition (3-11)) was obtained.

### [Experimental Example 5-2]

### (Evaluation of content proportions of compound (6-1-1), compound (6-2-1), compound (6-3-1), and impurities included in composition (3-1) to composition (3-11))

The compositions including the compound (6-1-1), the compound (6-2-1), and the compound (6-3-1) (composition (3-1) to composition (3-11)) were separated and analyzed by UHPLC (CAD) using a column (C18 column, 1.7 µm, inner diameter: 2.1 mm × 150 mm) and an eluent (continuous gradient of a 20 mM aqueous solution of ammonium acetate and a 20 mM ethanol solution of ammonium acetate. The evaluation results are illustrated in the following Table 2.

**[Table 2]**

| Solvent used for purification | Name of composition | UHPLC (CAD) area ratio (%) | | | |
|---|---|---|---|---|---|
| | | Compound (6-1-1) | Compound (6-2-1) | Compound (6-3-1) | Impurities |
| Cyclohexanone | (3-1) | 97.8 | 0.0 | 1.0 | 1.2 |
| Methyl isobutyl ketone | (3-2) | 95.2 | 0.2 | 2.2 | 2.4 |
| Diisopropyl ketone | (3-3) | 90.9 | 0.2 | 1.0 | 7.9 |
| Ethyl acetate | (3-4) | 91.7 | 0.3 | 0.0 | 8.0 |
| Butyl acetate | (3-5) | 93.0 | 0.0 | 5.1 | 1.9 |
| Diethyl ether | (3-6) | 96.5 | 0.1 | 0.5 | 2.9 |
| Dipropyl ether | (3-7) | 93.8 | 0.2 | 0.4 | 5.6 |
| Cyclopentyl methyl ether | (3-8) | 98.4 | 0.0 | 0.1 | 1.5 |
| Propylene glycol monomethyl ether acetate | (3-9) | 78.9 | 0.2 | 13.4 | 7.5 |
| Hexane | (3-10) | 85.7 | 0.2 | 0.8 | 13.3 |
| 2-Butanol | (3-11) | 57.4 | 0.0 | 0.0 | 42.6 |

### [Experimental Example 6]

### (Production of lipid nanoparticles encapsulating siRNA)

A lipid solution obtained by dissolving 1.83 mg of the composition (3-1), 0.158 mg of a phospholipid (DSPC, Catalog No. "COATSOME MC-8080", Yuka Sangyo Co., Ltd.), 0.696 mg of cholesterol (Catalog No. "C8667", Sigma-Aldrich Corporation), and 0.0502 mg of a PEG lipid (PEG-DMG, product name "SUNBRIGHT GM-020", Yuka Sangyo Co., Ltd.) in 0.5 mL of ethanol was mixed with 1.5 mL of an siRNA solution obtained by dissolving 0.0594 mg of an siRNA in an acetate buffer solution (pH 4) using a flow reactor. The solution after mixing was dialyzed with phosphate-buffered saline (PBS), and lipid nanoparticles (LNP1) encapsulating the siRNA were produced. As the siRNA, a product obtained by hybridizing a nucleic acid having a base sequence set forth in SEQ ID NO:1 and a nucleic acid having a base sequence set forth in SEQ ID NO:2 was used. This siRNA was to target the mRNA of mouse blood coagulation factor VII.

Lipid nanoparticles (LNP2 to LNP11) encapsulating the siRNA were produced by a similar method, except that the compositions (3-2) to (3-11) were used instead of the composition (3-1).

### [Experimental Example 7]

### (In vivo evaluation of in vivo kinetics of lipid nanoparticles encapsulating siRNA)

The in vivo kinetics of the lipid nanoparticles encapsulating an siRNA was evaluated. Specifically, (LNP1 to LNP11) produced in Experimental Example 6 were each administered to mice (C57BL/6NCrSlc, 6 weeks old, female, Sankyo Labo Service Corporation) through the tail vein such that the amount of the siRNA was 0.03 mg/kg of body weight. Subsequently, the mice were euthanized 24 hours later, and the liver and the spleen were extracted and frozen using liquid nitrogen.

Subsequently, each of the extracted organs was diluted with 0.25% Triton X-100-containing PBS and homogenized with a bead-type shredder. Subsequently, the resultant was treated at 95°C for 10 minutes to thermally denature proteins. Subsequently, each sample was left to stand on ice for 5 minutes and then centrifuged at 20,000 × g at 4°C for 20 minutes. Subsequently, the supernatant of each sample was treated at 95°C for 10 minutes, and a reverse transcription reaction of RNA was performed. A commercially available kit (product name "TaqMan MicroRNA Reverse Transcription Kit", Thermo Fisher Scientific, Inc.) was used for the reverse transcription reaction. The reaction conditions were 16°C for 30 minutes, subsequently 42°C for 30 minutes, and subsequently 85°C for 5 minutes.

Subsequently, a PCR reagent (product name "TaqMan Universal Master Mix II, no UNG", Thermo Fisher Scientific, Inc.) was added to each sample after the reverse transcription reaction, and the amount of siRNA in the liver and the spleen was quantified by real-time PCR.

Subsequently, the in vivo kinetics of each lipid nanoparticle was evaluated in vivo according to the following evaluation criteria. The results are shown in the following Table 3. The in vivo kinetics was evaluated by making a comparison with LNP9, which had the lowest delivery amount of siRNA into the liver and the spleen among LNP1 to LNP9.

### (Evaluation criteria)

++: The total amount of siRNA in the liver and the spleen was 65% or more of that of LNP9.
-: The total amount of siRNA in the liver and the spleen was less than 65% of that of LNP9.

### [Experimental Example 8]

### (Evaluation of in vivo knockdown activity of lipid nanoparticles encapsulating siRNA)

The in vivo knockdown activity of the lipid nanoparticles encapsulating an siRNA was evaluated. Specifically, (LNP1 to LNP11) produced in Experimental Example 6 were each administered to mice (C57BL/6NCrSlc, 6 weeks old, female, Sankyo Labo Service Corporation) through the tail vein such that the amount of the siRNA was 0.03 mg/kg of body weight. Subsequently, the mice were euthanized 24 hours later, and blood was collected through the inferior vena cava. Subsequently, the blood sample was centrifuged at 800 × g and 4°C for 5 minutes, and a blood plasma sample was obtained. Subsequently, using a BIOPHEN FVII Kit (Hyphen Biomed SAS), the amount of blood coagulation factor FV11 in each sample and the amount of blood coagulation factor FVII in the lipid nanoparticle-unadministered group were measured, and the knockdown rate was calculated on the basis of the following Expression (1). Knockdown rate (%) = (1 - (amount of blood coagualation factor FVII in sample/amount of blood coagulation factor FVII in lipid nanoparticle-unadministered group)) × 100

Subsequently, the knockdown activity of each lipid nanoparticle was evaluated according to the following evaluation criteria. The results are shown in the following Table 3.

### (Evaluation criteria)

++: Knockdown rate was 70% or more.
+: Knockdown rate was 30% or more and less than 70%.
-: Knockdown rate was less than 30%.

**[Table 3]**

| Solvent used for purification | Name of composition | Name of lipid nanoparticle | In vivo kinetics | Knockdown activity |
|---|---|---|---|---|
| Cyclohexanone | (3-1) | LNP 1 | ++ | ++ |
| Methyl isobutyl ketone | (3-2) | LNP 2 | ++ | ++ |
| Diisopropyl ketone | (3-3) | LNP 3 | ++ | ++ |
| Ethyl acetate | (3-4) | LNP 4 | ++ | ++ |
| Butyl acetate | (3-5) | LNP 5 | ++ | ++ |
| Diethyl ether | (3-6) | LNP 6 | ++ | ++ |
| Dipropyl ether | (3-7) | LNP 7 | ++ | ++ |
| Cyclopentyl methyl ether | (3-8) | LNP 8 | ++ | ++ |
| Propylene glycol monomethyl ether acetate | (3-9) | LNP 9 | ++ | ++ |
| Hexane | (3-10) | LNP 10 | - | ++ |
| 2-Butanol | (3-11) | LNP 11 | - | - |

### [Industrial Applicability]

According to the present invention, a technology is provided for removing impurities included in a composition including a lipid.

## Claims

1. A method for purifying a composition, the method comprising:
a step of dissolving a composition including a compound represented by the following Formula (1) in an aqueous layer to perform liquid-liquid extraction and refining the compound represented by the following Formula (1),
wherein an oil layer used for the liquid-liquid extraction includes one or more kinds of liquids selected from the group consisting of a ketone-based liquid, an ester-based liquid, and an ether-based liquid, all of which have a solubility parameter (SP value) of 14.8 to 20.5 (MPa^{1/2}), 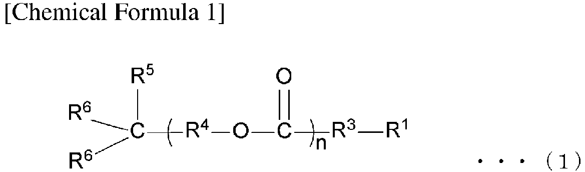 in the Formula (1), R¹ represents -N(R²)-R² (where each R² independently represents a C1 to C4 alkyl group), R³ represents a C3 to C8 alkanediyl group, R⁴ represents a C3 to C8 alkanediyl group, R⁵ represents a hydroxyl group; each R⁶ independently represents -R⁷-OH (where R⁷ represents a C4 to C12 alkanediyl group) or a hydrogen atom, and n represents an integer of 0 or 1.

2. The method for purifying according to Claim 1,
wherein the ketone-based liquid is cyclohexanone, methyl isobutyl ketone, or diisopropyl ketone, the ester-based liquid is ethyl acetate or butyl acetate, and the ether-based liquid is diethyl ether, dipropyl ether, cyclopentyl methyl ether, or propylene glycol monomethyl ether acetate.

3. A composition comprising:
one or more kinds of compounds selected from the group consisting of
a compound (2-1) in which, in a compound represented by the following Formula (2), n is 0, both R⁹'s are -R⁷-O-C(O)-R¹⁰, and R⁵ is a hydroxyl group,
a compound (2-2) in which, in a compound represented by the following Formula (2), n is 0, one R⁹ is -R⁷-O-C(O)-R¹⁰, the other R⁹ is -O-C(O)-R¹⁰, and R⁵ is a hydrogen atom, and
a compound (2-3) in which, in a compound represented by the following Formula (2), n is 1, both R⁹'s are -R⁷-O-C(O)-R¹⁰, and R⁵ is a hydroxyl group,
wherein a total content proportion of the compound (2-1), the compound (2-2), and the compound (2-3) included in the composition is 90% by mass or more, 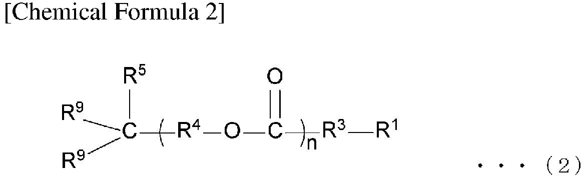 in the Formula (2), R¹ represents -N(R²)-R² (where each R² independently represents a C1 to C4 alkyl group), R³ represents a C3 to C8 alkanediyl group, R⁴ represents a C3 to C8 alkanediyl group, R⁵ represents a hydroxyl group or a hydrogen atom, each R⁹ independently represents -R⁷-O-C(O)-R¹⁰ or -O-C(O)-R¹⁰ (where R⁷ represents a C4 to C12 alkanediyl group, and R¹⁰ represents a C4 to C25 alkenyl group), where at least one R⁹ is -R⁷-O-C(O)-R¹⁰, and n represents an integer of 0 or 1.

4. The composition according to Claim 3,
wherein a content proportion of the compound (2-1) included in the composition is 85% to 99% by mass.

5. The composition according to Claim 3 or 4, wherein a total content proportion of the compound (2-2) and the compound (2-3) included in the composition is 0.1% to 15% by mass.

6. A compound in which, in the Formula (2) according to Claim 3, n is 0, one R⁹ is -R⁷-O-C(O)-R¹⁰, the other R⁹ is -O-C(O)-R¹⁰, and R⁵ is a hydrogen atom.

7. A compound in which, in the Formula (2) according to Claim 3, n is 1, both R⁹'s are -R⁷-O-C(O)-R¹⁰, and R⁵ is a hydroxyl group.

8. A lipid nanoparticle formed from a compound included in the composition according to Claim 3 or 4 and encapsulating a drug.

9. A lipid nanoparticle formed from a compound included in the composition according to Claim 5 and encapsulating a drug.

10. The lipid nanoparticle according to Claim 8,
wherein the drug is an siRNA.

11. The lipid nanoparticle according to Claim 9,
wherein the drug is an siRNA.
